# FASCICULE DE BREVET EUROPEEN

(11) **EP 0 506 769 B1**
(45) Date de publication et mention de la délivrance du brevet: **27.07.1994**
(21) Numéro de dépôt: 91901458.9
(22) Date de dépôt: 18.12.1990
(51) Int. Cl.: A61K 37/64, A61K 37/02, A61K 31/19, A61K 31/16

(54) **COMPOSITION PHARMACEUTIQUE DESTINEE AU TRAITEMENT OU A LA PREVENTION DES INFECTIONS RETROVIRALES**
PHARMAZEUTISCHE ZUSAMMENSETZUNG BESTIMMT ZUR BEHANDLUNG ODER VORBEUGUNG VON RETROVIRALEN INFEKTIONEN
PHARMACEUTICAL COMPOSITION FOR TREATING OR PREVENTING RETROVIRAL INFECTIONS

(30) Priorité: 18.12.1989 FR 8916726; 23.10.1990 FR 9013102
(43) Date de publication de la demande: 07.10.1992
(73) Titulaire: TRANSGENE S.A., 67082 Strasbourg Cédex (FR)
(72) Inventeur: GUY, Bruno, F-67000 Strasbourg (FR); LECOCQ, Jean-Pierre, F-67116 Reichstett (FR)
(74) Mandataire: Warcoin, Jacques
(86) Numéro de dépôt international: FR9000921
(87) Numéro de publication internationale: WO9108769

(56) Documents cités:
- EP-A- 0 188 262
- EP-A- 0 269 311
- Aids Research and Human Retroviruses, vol. 5, no. 3, Juin 1989, Mary Ann Liebert Inc., Publishers, B.Grinde et al., p. 269-274
- Journal of Virology, vol. 48, no. 3, December1983, American Society for Microbiology, K.B.Andersen, p. 765-769
- Int. J.Cancer, vol. 27, 1981, K.B.Hellman et al., p. 95-99.
- Thrombosis Research, vol. 37, 1986, Pergamon Press Ltd., (US), G.Mitra et al, p. 291-300
- Molecular and Biochemical Parasitology, vol. 35, 1989, Elsevier Science Publishers B.V. P.J.Rosenthal et al, p. 177-184

## Description

La présente invention concerne une composition pharmaceutique destinée au traitement des infections rétrovirales, et notamment du syndrome d'immunodéficience acquise (SIDA).

Le SIDA est une infection virale qui se développe de façon importante en Amérique du Nord, en Europe et en Afrique. La recherche d'un vaccin ou d'un traitement est l'objectif majeur d'un grand nombre d'équipes de recherche de part le monde.

Le rétrovirus HIV (Human Immunodeficiency Virus), qu'il s'agisse de HIV1 ou de HIV2 a été reconnu comme l'agent responsable du SIDA chez l'homme. Outre le virus HIV, il existe d'autres rétrovirus qui induisent une pathologie similaire dans diverses espèces animales : par exemple, le virus ovin Visna, le virus SIV (Simian Immunodeficiency Virus), le virus FIV (Feline Immunodeficiency Virus) ou le virus BIV (Bovine Immunodeficiency Virus).

Brièvement, la structure des rétrovirus se présente de la manière suivante : une nucléocapside comprenant la molécule d'ARN génomique associée à diverses protéines et protégée par une capside de nature protéique, l'ensemble étant enveloppé dans une membrane d'origine cellulaire ayant cependant incorporé des protéines virales.

Les gènes gag, pol et env sont caractéristiques des rétrovirus. Le gène gag code pour un précurseur qui est ensuite clivé pour donner plusieurs protéines composant la nucléocapside. Le gène pol code pour un précurseur qui est ensuite clivé pour donner la protéase, la transcriptase inverse qui transcrit, dans la cellule infectée, l'ARN viral en ADN et l'intégrase qui permet l'intégration de cet ADN dans le génome de la cellule hôte. Le gène env code pour un précurseur de la protéine d'enveloppe du virus qui est glycosylé puis clivé en une grande sous-unité et une petite sous-unité. Lors de la multiplication du virus dans les cellules infectées, la petite sous-unité s'ancre dans la membrane cellulaire par son domaine transmembranaire. Sa partie C-terminale reste en contact avec le cytoplasme et constitue ainsi le domaine intra-cytoplasmique tandis que sa partie N-terminale se trouve à la surface de la cellule et constitue ainsi le domaine extracellulaire. La grande sous-unité est relarguée à l'extérieur de la cellule où elle interagit avec le domaine extracellulaire de la petite sous-unité. Les deux sous-unités de la protéine d'enveloppe restent ainsi associées sous forme de complexe. Lorsque les particules virales se forment, elles bourgeonnent à la surface de la cellule et s'enveloppent ainsi dans la membrane cellulaire dans laquelle sont ancrées des molécules de la protéine d'enveloppe du virus.

Dans le cas du virus HIV1, le gène env code pour une glycoprotéine précurseur gp 160 qui est clivée en une petite sous-unité gp 41 et en une grande sous-unité gp 120.

Outre les gènes gag, pol, et env, il existe d'autres gènes communs à certains rétrovirus, notamment aux rétrovirus responsables des syndromes d' immunodéficience acquise. Parmi ceux-ci se trouve le gène vif codant pour un facteur déterminant le pouvoir infectieux du virus (vif : virion infectivity factor). Ce facteur d'un PM de 20 à 30 kDa (23 kDa pour le HIV1) est détecté dans le cytoplasme des cellules infectées. Du expériences ont montré que les virions provenant de souches comportant des mutations qui inactivent la protéine vif, ont des ARN et du enzymes de réplication normaux, mais qu'ils sont de 100 à 1000 fois moins infectieux.

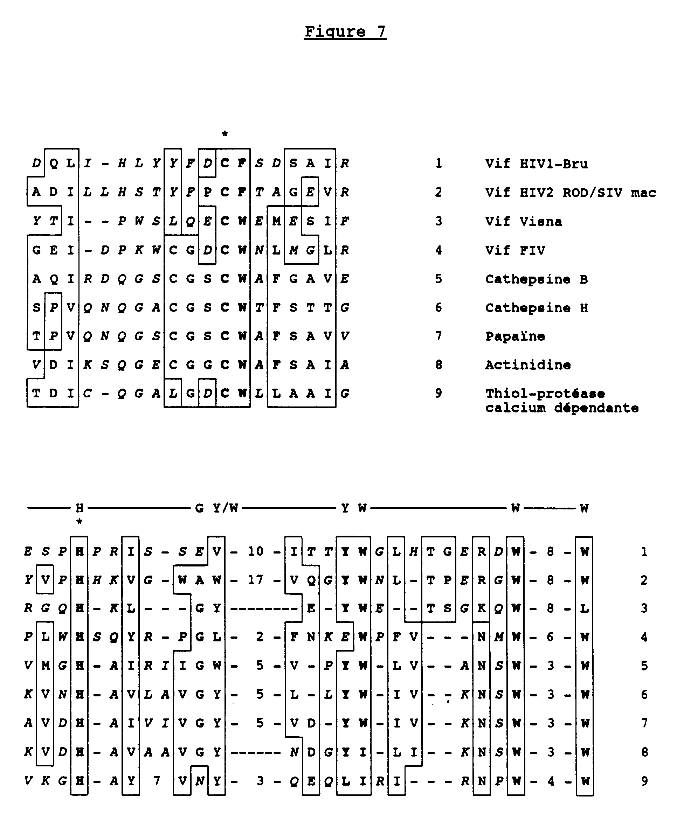

D'une manière générale, différents groupes de recherche ont constaté que les protéines vif issues de différents virus de type HIV (HIV1, HIV2, SIV, FIV, Visna) ne présentaient pas d'homologie significative entre elles (S. Wain-Hobson et al. Cell (1985), 40, 9-17; P. Sonigo et al. Cell (1985), 42, 369-382; R. A. Olmsted et al. Proc. Natl. Acad. Sci. USA (1989), 86, 8088-8092; L. Chakrabarti et al. Nature (1987), 328, 543-547 ; M. Guyader et al. Nature (1987), 326, 662-669). Cependant dans ces protéines vif, on a maintenant mis en évidence de très courtes séquences qui présentent un degré d'homologie entre elles, par identité ou équivalence des acides aminés. De plus, certaines de ces séquences ne se retrouvent que dans des protéines ayant une activité thiol-protéase, par exemple la cathepsine B ou H la papaïne et l'actinidine. Pour illustration, voir Figure 7.

EP 269 311 a pour objet le traitement de maladies osseuses de nature non infectieuse avec la cystatine.

EP 188 262 divulgue l'usage de cystatine isolée du blanc d'oeuf pour traiter des infections à picornavirus.

L'article de Journal of Virology, vol. 48(3) décrit l'utilisation de la leupeptine contre un rétrovirus murin qui ne possède pas la protéine vif.

Ainsi, il a maintenant été trouvé qu'une activité thiol-protéase est associée à la protéine vif, et que cette activité a pour substrat la petite sous-unité de la protéine d'enveloppe. La protéine vif interviendrait donc dans le processus de maturation de la protéine d'enveloppe en agissant sur sa structure et/ou son agencement, avec pour conséquence un haut degré d'infectivité des virions.

En conséquence, la présente invention propose:
(i) l'utilisation d'un inhibiteur de thiol-protéase pour l'obtention d'un médicament destiné au traitement ou à la prévention des infections à rétrovirus d'immunodéficience acquise, et
(ii) l'utilisation d'un inhibiteur de thiol-protéase pour l'obtention d'un médicament destiné au traitement ou à la prévention du SIDA.

Parmi les infections à rétrovirus en cause, il faut citer plus particulièrement les infections à virus de type HIV et notamment les infections à HIV-1.

Les inhibiteurs de thiol-protéases sont du produits connus ; par exemple les cystatines et leurs dérivés, l'antipaïne [(S)-1-carboxy-2-phenylethyl]carbamoyl-Arg-Val-Arg-al, E-64 (L-trans-epoxysuccinyl-leucylamide-(4-guanidino)-butane, N-[N-(L-3-transcarboxyirane-2-carbonyl)-L-leucyl]-agmatine, la leupeptine, l'acide iodoacétique/iodoacétamide ou le N-Cbz-Phe-Ala-diazométhane.

Néanmoins, aux fins de la présente invention, des composés plus particulièrement préférés sont du inhibiteurs spécifiques de la protéine vif. Ce dernier type d'inhibiteurs inclut, par exemple, des oligopeptides dont la structure tridimensionnelle reproduit celle d'un site de reconnaissance de la protéine vif localisé sur la petite sous-unité de la protéine d'enveloppe.

L'activité d'un inhibiteur de thiol-protéases utilisé aux fins de la présente invention peut être mise en évidence dans un test in vitro mettant en oeuvre une cible appropriée ; par exemple, le virus HIV ou un double système d'expression env/vif.

Connaissant les particularités du SIDA, les doses appliquées peuvent varier dans une large mesure et dépendent de l'état du sujet traité et de l'activité du composé particulier mis en oeuvre.

La protéine vif étant localisée dans le cytoplasme des cellules infectées, les inhibiteurs de thiol-protéases doivent pouvoir pénétrer à l'intérieur de la cellule pour pouvoir atteindre vif. Pour cela il convient de choisir des inhibiteurs capables de pénétrer dans la cellule, modifiés ou couplés à d'autres molécules ou encore inclus dans des liposomes. De préférence, on choisira un système permettant aux inhibiteurs de thiol-protéases de n'agir que sur les cellules infectées.

Les formes galéniques utilisables sont très variées, il peut s'agir de formes injectables ou non, dont l'homme du métier connaît les caractéristiques.

Les exemples ci-après sont destinés à mettre en évidence d'autres caractéristiques et avantages de la présente invention et font référence aux figures suivantes :

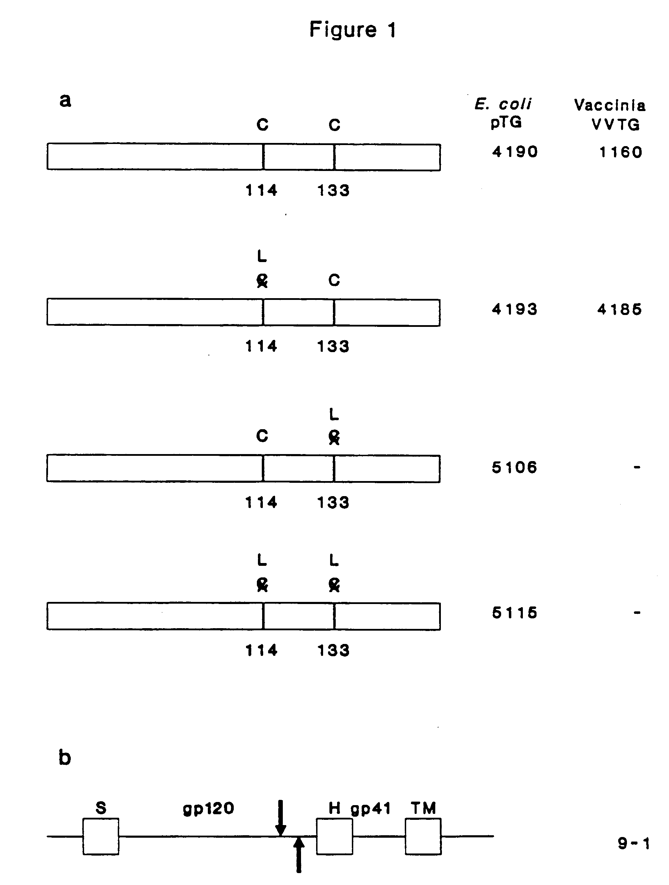

La Figure 1 représente de façon schématique les fragments d'ADN codant pour la protéine vif forme sauvage ou formes mutées insérés dans des plasmides d'expression chez *E. coli* et dans le génome du virus de la vaccine (figure 1a) et, le fragment d'ADN codant pour la protéine env inséré dans génome du virus de la vaccine; S représente la séquence signal, H représente la séquence hydrophobe et TM représente la séquence transmembranaire. Les flêches indiquent les sites de coupures du précurseur pour donner la gp120 et la gp41 (figure 1b).

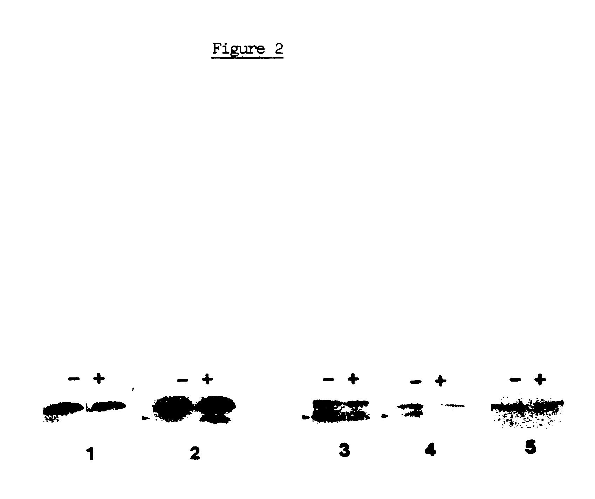

La Figure 2 représente la portion aux alentours de 20-25 Kda d'une autoradiographie d'une électrophorèse en gel de polyacrylamide (30mA) de surnageants de sonication marqués par de l'iodoacétamide après incubation en présence (+) ou absence (-) de E64 10 µM de souches d'*E. coli* TGE901 transformées par les plasmides suivants :
   - colonnes 1⁻ et 1⁺ : pTG959, contrôle négatif
   - colonnes 2⁻ et 2⁺ : pTG4190 exprimant la protéine vif de forme sauvage
   - colonnes 3⁻ et 3⁺ : pTG4193 exprimant la protéine vif mutée en position 114
   - colonnes 4⁻ et 4⁺ : pTG5106 exprimant la protéine vif mutée en position 133
   - colonnes 5⁻ et 5⁺ : pTG5115 exprimant la protéine vif mutée en positions 114 et 133.

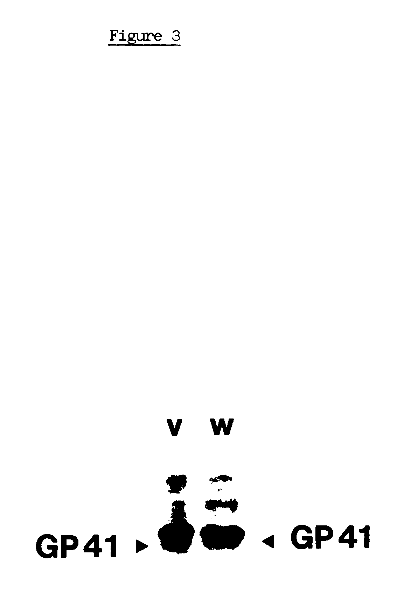

La Figure 3 représente la portion aux alentours de 35-45 Kda d'une autoradiographie d'une électrophorèse en gel de polyacrylamide (5mA; tampon de charge conventionnel supplémenté avec 200 mM de phospate de sodium) des échantillons décrits ci-après, immunoprécipités par un sérum humain HIV positif. Les échantillons sont des extraits cellulaires (P) ou des surnageants (S) d'une culture de cellules BHK-21 (W)coinfectées par le virus de la vaccine VVTG9-1 (exprimant la protéine env) et par le virus de la vaccine forme sauvage VVWT (contrôle) et d'une culture de cellules BHK-21 (V) coinfectées par le virus de la vaccine VVTG9-1 et par le virus de la vaccine VVTG1160 (exprimant la protéine vif de forme sauvage). La flêche indique la gp41.

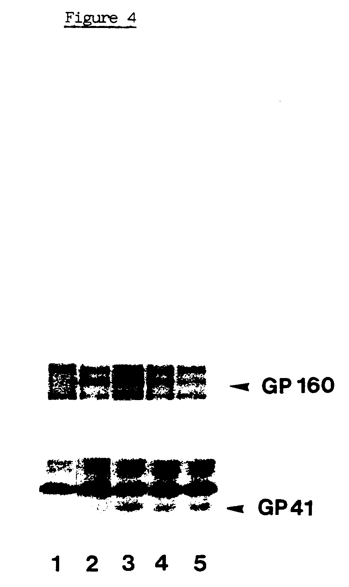

La Figure 4 représente les portions aux alentours de 100-200 kDa et 40-45 Kda d'une autoradiographie d'une électrophorèse en gel de polyacrylamide (30 mA) d'extraits de cellules BHK-21 immunoprécipités par un sérum polyclonal de lapin dirigé contre la partie C-terminale de la gp41. Les cellules BHK-21 infectées par VVWT (colonne 1) ou co-infectées par VVTG9-1 (exprimant la protéine env) et VVTG1160 (exprimant la protéine vif de forme sauvage) [colonne 2 et 5] ou par VVTG9-1 et VVWT [colonne 3] ou par VVTG9-1 et VVTG4185 (exprimant la protéine vif mutée en position 114) sont cultivées en absence de E64-D [colonnes 1 à 4] ou en présence de E64-D 10 µM [colonne 5].

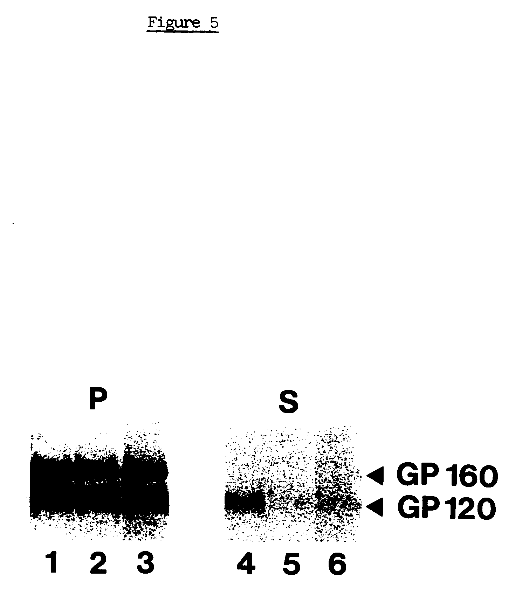

La Figure 5 représente la portion aux alentours de 100-200 kDa d'une autoradiographie d'une électrophorèse en gel de polyacrylamide (30 mA) d'extraits de cellules BHK-21 [colonnes 1 à 3] ou de surnageants [colonnes 4 à 6] immunoprécipités par un sérum humain HIV positif. Les cellules BHK-21 co-infectées par VVTG9-1 (exprimant la protéine env) et VVWT [colonnes 1 et 4] ou par VVTG9-1 et par VVTG1160 (exprimant la protéine vif de forme sauvage) [colonnes 2,3,5 et 6] sont cultivées en absence de E64-D [colonnes 1,2,4 et 5] ou en présence de E64-D 10 µM [colonnes 3 et 6]. Les flêches indiquent la gp160 et la gp120.

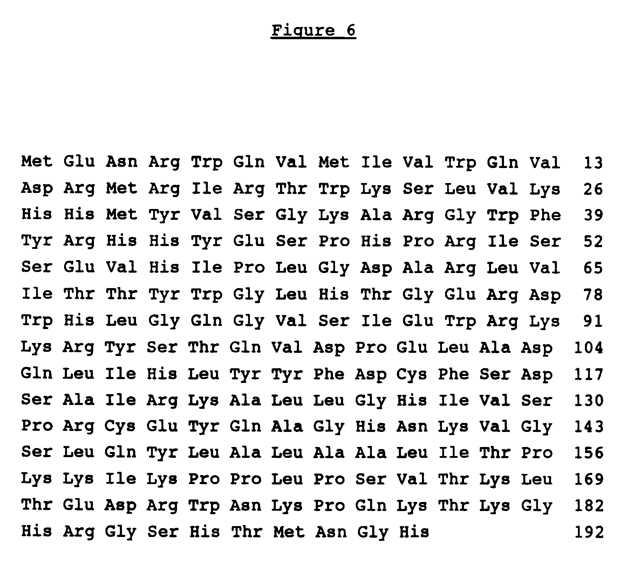

La Figure 6 représente la séquence en acides aminés de la protéine vif de HIV1, isolat Bru, telle que initialement synthétisée à partir d'un ARNm. Le résidu méthionine n°1 en position N-terminale est normalement éliminé par la suite.
La Figure 7 présente les régions homologues (par identité ou équivalence des acides aminés) des protéines vif des virus HIV1-Bru, HIV2 ROD/SIV mac, Visna et FIV ainsi que celles de différentes thiol-protéases. Les acides aminés non conservés sont indiqués en italiques. Les chiffres placés dans les séquences indiquent le nombre d'acides aminés présents.

### EXEMPLE 1 : Mise en évidence d'une fonction thiol-protéase associée à la protéine vif.

Les thiol-protéases possédent un résidu cystéine dans leur site actif. De plus il est connu que les thiol-protéases peuvent être spécifiquement inhibées par le E64. Lorsqu'une thiol-protéase est mise en contact avec du E64, celui-ci se fixe sur le site actif et bloque le résidu cystéine de ce site.

Pour mettre en évidence un site actif caractéristique d'une thiol-protéase on effectue un test d'inhibition du marquage à l'iodoacétamide par le E64, l'iodoacétamide se fixant de manière non spécifique sur les cystéines de la plupart des protéines.

Enfin pour identifier avec précision la position du site actif dans la protéine vif, on teste la protéine vif de forme sauvage ainsi que des protéines vif mutées à l'emplacement des résidus cystéine originaux en positions 114 et 133.

### 1A. Expression de la protéine vif de forme sauvage dans E. coli

Le fragment BglII-EcoRI du phage M13TG185 décrit par Y. Rivière et al., J. Virol. (1989) 66 : 2270, qui comporte la séquence d'ADN codant pour la protéine vif de HIV1, isolat Bru, est inséré dans le plasmide pTG959 (décrit dans la demande de brevet EP-A-292 404) digéré par les enzymes BglII et EcoRI pour donner le plasmide pTG4190, dans lequel la séquence d'ADN codant pour la protéine vif est sous le contrôle du promoteur pL du phage lambda.

La souche TGE901 (décrite sous la dénomination N4830/pKC30 dans Gottesman M.E. et al., J Mol. Biol. (1980) 140 : 57) est transformée par le plasmide pTG4190 puis cultivée à 30°C jusqu'à une DO₆₀₀ de 0,1. L'expression du fragment d'ADN codant pour la protéine vif est induite à 42°C pendant 4 heures. Les cellules sont récoltées par centrifugation et resuspendues dans 20 ml de PBS pour 500 ml de culture, puis elles sont soniquées 8 min. dans un bain de glace plus alcool. La solution est alors centrifugée, et le surnageant contenant environ 1% de la protéine vif exprimée est conservé pour effectuer le test.

### 1B. Expression dans E. coli des protéines vif mutées en positions 114 ou 133 ou 114 et 133.

Le phage M13TG185 est soumis à une mutagénèse dirigée, ciblée sur le codon du fragment BglII-EcoRI correspondant à l'acide aminé en position 114 de la protéine vif, en utilisant la trousse Amersham et l'oligonucléotide OTG2540 :
5' GCAGAGTCTGAAAAGAGCTCAAAGTAATACAG 3'. Ceci correspond à la substitution de la cystéine originelle en position 114 par une leucine. Le fragment BglII-EcoRI muté est alors inséré dans le plasmide d'expression pTG959 préalablement digéré par les enzymes BglII et EcoRI, pour donner le plasmide pTG4193 (Figure 1).

De la même façon la cystéine en position 133 est mutée en leucine en utilisant l'oligonucléotide OTG2658 :
5' GCTTGATATTCAAGCTTAGGGCTAACT 3'. Le fragment BglII-EcoRI ainsi muté est inséré dans le plasmide pTG959 digéré par les enzymes BglII et EcoRI pour donner le plasmide pTG5106 (Figure 1).

De la même façon les cystéines en positions 114 et 133 sont simultanément mutées en leucine en utilisant les oligonucléotides OTG2540 et OTG2658. Le fragment BglII-EcoRI ainsi muté est inséré dans le plasmide pTG959 digéré par les enzymes BglII et EcoRI pour donner le plasmide pTG5115 (Figure 1).

La souche *E. coli* TGE901 est transformée par les plasmides pTG4193, pTG5106 ou pTG5116. La culture et la sonication des bactéries s'effectuent comme décrit au paragraphe 1A, et les surnageants de sonication sont récupérés de même.

### 1C. Tut d'inhibition du marquage à l'iodoacétamide par le E64 :

Deux échantillons de 40 µl de chaque surnageant obtenu en 1A et 1B sont incubés dans du tampon PBS (Dubelcco's phosphate buffer salt ; Seromed) pendant 30 min. à 37°C, un des échantillons étant préalablement supplémenté avec du E64 10 µM (Sigma). Les 2 échantillons sont alors incubés dans du tampon PBS en présence de 10 µM d'iodoacétamide marquée au C¹⁴ (Amersham) pendant 30 min. à 37°C. Un contrôle négatif est effectué sur un surnageant de sonication obtenu après culture de la souche de *E. coli* TGE901 transformée par le plasmide pTG959. Ces échantillons sont soumis à une électrophorèse en gel SDS-polyacrylamide (Figure 2).

La protéine vif sauvage, marquée par l'iodoacétamide, migre sous la forme d'une large bande. Une préincubation avec du E64 modifie la migration apparente de la protéine vif, ce qui démontre que le E64 peut former un complexe avec la protéine vif.

La protéine vif mutée en position 133, et qui donc ne possède que la cystéine 114, n'est plus marquée par l'iodoacétamide lorsqu'elle est préincubée avec du E64. A l'inverse la préincubation avec le E64 n'inhibe pas le marquage par l'iodoacétamide de la protéine vif mutée en position 114, qui ne possède plus que la cystéine 133. La protéine vif mutée sur les deux cystéines 114 et 133 n'est pas marquée par l'iodoacétamide.

Le test d'inhibition précédemment décrit est renouvelé en remplaçant le E64 par un inhibiteur de sérine protéases tels que le Phénylméthane-sulfonylfluoride ou le Soy Trypsine Inhibiteur 100 µM. Un tel inhibiteur ne modifie pas le marquage de la protéine vif qu'elle soit sous forme sauvage ou mutée.

Ces résultats montrent que :
- la protéine vif de HIV1 posséde un domaine ayant une structure comparable au site actif des thiol-protéases et que,
- la cystéine en position 114 fait partie de ce domaine.

### EXEMPLE 2 : Mise en évidence de l'effet inhibiteur du E64 sur la maturation de la protéine gp41 du virus HIV1.

La protéine vif sous forme sauvage ou mutée en position 114 est co-exprimée avec la protéine env dans les cellules BHK-21 (Baby Hamster Kidney) de la manière suivante :
Tout d'abord les vecteurs d'expression dans les cellules BHK-21, des différentes formes de la protéine vif et de la protéine env sont les suivants :
Le virus de la vaccine recombinant VVTG1160 comportant le fragment d'ADN codant pour la protéine vif de forme sauvage est décrit par Y. Rivière et al. (1989), J. Virol. 63, pp. 2270-2277.

Le virus de la vaccine recombinant VVTG4185 comportant le fragment d'ADN codant pour la protéine vif mutée en position 114 est obtenu par insertion du fragment BglII-EcoRI du plasmide pTG4193 dans le génome du virus de la vaccine, selon la technique décrite par M.P. Kieny et al. (1984), Nature 312. pp. 163-166.

Le virus de la vaccine recombinant VVTG9-1 comportant le fragment d'ADN codant pour la protéine env native de HIV1, isolat Bru, est décrit dans Kieny M.P. et al. (1986), Biotechnology 4, pp. 790-795.

Tous les virus de la vaccine recombinants dérivent du virus de la vaccine, souche Copenhagen.

10⁶ cellules BHK-21 sont co-infectées par (i) VVTG9-1 (env) et VVTGWT (virus de la vaccine souche Copenhagen type sauvage : Wild Type), qui sert de contrôle ou (ii) par VVTG9-1 (env) et VVTG1160 (vif de forme sauvage) ou (iii) par VVTG9-1 et VVTG4185 (vif muté en position 114). L'infection s'effectue à une multiplicité de 10 ufp dans le cas de VVTG9-1 et de 15 ufp dans les autres cas.

Après une heure d'adsorption des virus à 20 °C, les cellules BHK-21 sont mises en culture dans un milieu MEM sans méthionine (Gibco), supplémenté avec 100 µCi de méthionine S³⁵ (Amersham), en présence ou absence de 10 à 100 µM de E64-D (ethyl(25,35)-3[(5)-3-methyl-1-(3-methylbutylcarbamoyl)butylcarbamoyl] oxirane-2-carboxylate 1, un dérivé de E64 qui pénètre dans les cellules ; synthétisé par Neosystem, Strasbourg). Après 4 heures d'incubation à 37°C, la culture est centrifugée afin de récupérer séparément les cellules lysées et le surnageant. La présence de la protéine env est détectée dans le surnageant et dans le lysat cellulaire par immunoprécipitation avec un antisérum polyclonal humain HIV positif, selon la méthode décrite dans Kieny M.P. et al. (1984) cité ci-dessus. Le produit de l'immunoprécipitation est soumis à une électrophorèse sur un gel SDS-polyacrylamide (Figure 3 et 5). En parallèle, la présence de la protéine vif de forme sauvage ou mutée est mise en évidence dans les lysats cellulaires par la technique du western blot en utilisant un anticorps monoclonal de souris dirigé contre la protéine vif.

Tel que montré à la Figure 3, on remarque que, en présence de la protéine vif de forme sauvage, la mobilité relative de la protéine gp41 est sensiblement supérieure à celle de la gp41 présente dans les autres échantillons testés. Ceci correspondant à la perte d'environ 1 KDa.

De plus la protéine vif mutée en position 114 n'induit pas la modification post-traductionnelle de la gp41 observée en présence de la forme sauvage de la protéine vif, cette modification post-traductionnelle serait due à l'activité thiol-protéase associée à la protéine vif. Ceci est confirmé par le fait que le E64-D abolit l'effet de la protéine vif.

En conclusion, l'exemple 2 démontre qu'un inhibiteur des thiol-protéases tel que le E64 bloque la maturation de la gp41 en inhibant une fonction protéolytique associée à la protéine vif.

Les lysats cellulaires obtenus précédemment sont de même soumis à des tests d'immunoprécipitation avec un antisérum de lapin dirigé contre les 15 résidus d'acides aminés en position C-terminale communs aux protéines env et gp41 (fourni par Neosystem, Strasbourg). Le produit des immunoprécipitations est traité par électrophorèse sur gel de SDS-polyacrylamide tel que montré à la Figure 4. Quelque soit le type de coinfection des quantités similaires de précurseur gp160 sont immunoprécipitées. Par contre la quantité de gp41 est plus faible dans le cas d'une co-infection par VVTG9-1 et VVTG1160 que dans le cas d'une co-infection par VVTG9-1 et VVTG4185 ou par VVTG9-1 et VVWT. Comme la gp41 correspond au coté C-terminal du précurseur gp160, et compte tenu des observations précédentes, il semble que la protéine vif agisse spécifiquement sur la gp41, après le clivage du précurseur gp160 en gp120 et gp41.

Tel que montré à la figure 4, colonne 5, la présence de E64-D dans une culture des cellules co-infectées par VVTG9-1 et VVTG1160 abolit l'effet de vif. Aucune différence n'est observée dans le contrôle en présence ou absence de E64-D.

D'autre part la Figure 4 indique que la présence de E64-D dans une culture de cellules co-infectées par VVTG9-1 et VVTG1160 n'affecte pas la quantité de la gp160 immunoprécipitée. Comme le E64 est un inhibiteur de thiol-protéases non spécifique, ceci démontre bien que la modification post-traductionnelle de la gp41 n'est pas le fait de thiol-protéases intervenant dans le catabolisme cellulaire.

### EXEMPLE 3 : Effet du E64 sur la protéine env.

Les cellules BHK-21 sont co-infectées par VVTG9-1 (env) et VVTGWT ou par VVTG9-1 (env) et VVTG1160 (vif de forme sauvage) en présence ou absence de E64-D, comme décrit dans l'Exemple 2. Les lysats cellulaires et les surnageants ainsi obtenus sont soumis à des tests d'immunoprécipitation avec un antisérum polyclonal humain HIV positif.

Tel que montré à la Figure 5, en absence de E64-D, les quantités totales du précurseur gp160 et de la gp120 présents dans les lysats cellulaires sont identiques que vif soit synthétisé ou non (colonnes PV et PW), par contre la quantité de gp120 présente dans le surnageant est remarquablement supérieure quand env est co-exprimée avec vif (SV), par rapport au contrôle (SW). En présence de E64-D et quand vif est co-exprimée avec env, le relargage de gp120 dans le surnageant est similaire à celui observé dans le test contrôle (SW).

Quand vif est inhibé par le E64 on observe un effet indirect sur le comportement de la gp120 et, dans ce cas, la protéine env (complexe gp120-gp41) pourrait être déficiente. Ceci suggère qu'en présence de E64, les virions incorporeraient dans leurs capsides une protéine d'enveloppe défectueuse qui modifierait leur structure. Le E64 pourrait ainsi atténuer l'infectivité des virions.

### EXEMPLE 4

L'expression de la protéine env native dans des cellules BHK est obtenue en utilisant comme vecteur le virus de la vaccine recombinant VVTG9-1. L'expression de la protéine env fusionée à la glycoprotéine de la rage dans des cellules BHK est obtenue en utilisant comme vecteur le virus de la vaccine recombinant VVTG1131 décrit par Kieny et al., Protein Eng. (1986) 2: 210. L'expression de la protéine vif dans les cellules BHK est obtenue en utilisant comme vecteur le virus de la vaccine recombinant VVTG1160.

Les conditions d'infection sont les suivantes : 10⁶ cellules BHK 21 sont infectées avec une multiplicité de 10 ufp par cellule avec VVTG9-1 ou VVTG1131 exprimant la protéine env native ou fusionnée à la glycoprotéine de la rage, et/ou avec une multiplicité de 20 ufp avec VVTG1160 exprimant la protéine vif ou avec le virus de la vaccine type sauvage(VVWT).

Après une heure d'adsorption des virus, les cellules BHK sont mises en culture dans un milieu MEM sans méthionine, supplémenté avec 100 µCi de méthionine ³⁵S pour 10⁶ cellules. Après 5, 6 ou 7 heures de marquage, elles sont lysées et le culot et le surnageant sont sépares. L'expression de la protéine env est détectée par immunoprécipitation (marquage à la méthionine ³⁵S) avec un anticorps polyclonal humain HIV positif et celle de la protéine vif est mise en évidence par Western blot en utilisant un anticorps monoclonal anti-protéine vif de HIV-1.

Les expressions de la protéine env obtenues suite à une infection des cellules BHK par VVTG9-1 seul et suite à une infection par VVTG9-1 + VVWT sont identiques.

Lorsque seule la protéine env est exprimée, on détecte la protéine gp120 dans le surnageant où elle est relarguée après maturation complète. Lorsque l'on exprime à la fois la protéine env et la protéine vif, on observe deux types d'effets. Le premier correspond à une action de la protéine vif sur la traduction en général (effet qualifié de non-spécifique), diminuant le taux de synthèse de certaines protéines et augmentant celui d'autres protéines. Le second effet correspond à l'activité spécifique de la protéine vif sur les protéines de l'enveloppe. On observe une accélération de la maturation de la protéine gp160 ainsi qu'une augmentation de la quantité de la protéine gp120 présente à la surface de la cellule et dans le surnageant. D'autre part, lorsque l'on exprime la protéine de l'enveloppe dont la région trensmembranaire et la partie intra-cytoplasmique sont remplacées par la région transmembranaire et la partie intra-cytoplasmique de la glycoprotéine du virus de la rage, on n'observe plus d'action spécifique de la protéine vif.

Lorsque les mêmes expériences sont réalisées en présence d'un inhibiteur de thiol-protéases, l'iodoacétamide, à une concentration de 10 µM on ne retrouve quasiment plus de protéine gp120 dans le surnageant. Ceci suggère que la maturation de la protéine env est bloquée ou fortement ralentie. La présence de l'iodoacétamide inhibe aussi bien l'action non-spécifique de la protéine vif sur la traduction que son action spécifique sur la protéine de l'enveloppe.

## Revendications (Revendications pour l'(les) Etat(s) contractant(s) suivant(s): AT, BE, CH, DE, DK, FR, GB, IT, LI, LU, NL, SE)

1. Utilisation d'un inhibiteur de thiol-protéase pour l'obtention d'un médicament destiné au traitement ou à la prévention des infections à rétrovirus d'immunodéficience acquise.

2. Utilisation d'un inhibiteur de thiol-protéase pour l'obtention d'un médicament destiné au traitement ou à la prévention du SIDA.

## Revendications (Revendications pour l'(les) Etat(s) contractant(s) suivant(s): ES, GR)

1. Utilisation d'un inhibiteur de thiol-protéase en association avec un support ou un diluant acceptable d'un point de vue pharmaceutique, pour la préparation d'une composition pharmaceutique destinée au traitement ou à la prévention des infections à rétrovirus d'immunodéficience acquise.

2. Utilisation d'un inhibiteur de thiol-protéase en association avec un support ou un diluant acceptable d'un point de vue pharmaceutique, pour préparer une composition pharmaceutique destinée au traitement et à la prévention du SIDA.

## Claims (Claims for the following Contracting State(s): AT, BE, CH, DE, DK, FR, GB, IT, LI, LU, NL, SE)

1. Use of a thiol-protease inhibitor for producing a medicinal substance for treating or preventing acquired immuno-deficiency retroviral infections.

2. Use of a thiol-protease inhibitor for producing a medicinal substance for treating or preventing AIDS.

## Claims (Claims for the following Contracting State(s): ES, GR)

1. Use of a thiol-protease inhibitor in combination with a pharmaceutically acceptable carrier or diluent, for the preparation of a pharmaceutical composition for treating or preventing acquired inmmunodeficiency retroviral infections.

2. Use of a thiol-protease inhibitor in combination with a pharmaceutically acceptable carrier or a diluent, to prepare a pharmaceutical composition for treating and preventing AIDS.

## Patentansprüche (Patentansprüche für folgende(n) Vertragsstaat(en): AT, BE, CH, DE, DK, FR, GB, IT, LI, LU, NL, SE)

1. Verwendung eines Thiol-Protease-Inhibitors zur Herstellung eines Medikaments zur Behandlung oder Vorbeugung von retroviralen Infektionen bei erworbener Immundefizienz.

2. Verwendung eines Thiol-Protease-Inhibitors zur Herstellung eines Medikaments zur Behandlung oder Vorbeugung von AIDS.

## Patentansprüche (Patentansprüche für folgende(n) Vertragsstaat(en): ES, GR)

1. Verwendung eines Thiol-Protease-Inhibitors in Verbindung mit einem pharmazeutisch annehmbaren Träger oder Verdünner zur Herstellung einer pharmazeutischen Zusammensetzung zur Behandlung oder Vorbeugung von retroviralen Infektionen bei erworbener Immundefizienz.

2. Verwendung eines Thiol-Protease-Inhibitors in Verbindung mit einem pharmazeutisch annehmbaren Träger oder Verdünner zur Herstellung einer pharmazeutischen Zusammensetzung zur Behandlung oder Vorbeugung von AIDS.
